# EUROPEAN PATENT APPLICATION

(11) **EP 3 771 439 A1**
(43) Date of publication of application: **03.02.2021**
(21) Application number: 20188929.2
(22) Date of filing: 31.07.2020
(51) Int. Cl.: A61B 17/122, A61B 17/128

(54) **LIGATION CLIP WITH IMPROVED LATCH MECHANISM**

(30) Priority: 02.08.2019 US 201962881966 P; 13.05.2020 US 202015931096
(71) Applicant: Covidien LP, Mansfield, MA 02048 (US)
(72) Inventor: PILLETERE, Roy J., North Haven, CT Connecticut 06473 (US); THOMAS, Justin, New Haven, CT Connecticut 06512 (US); DININO, Matthew A., Newington, CT Connecticut 06111 (US); BANERJEE, Saumya, Hamden, CT Connecticut 06514 (US); MORCK, Gregory R., Haddam, CT Connecticut 06438 (US); BROWN, Eric, Madison, CT Connecticut 06443 (US)
(74) Representative: Maschio & Soames IP Ltd

(57) **Abstract**

A polymeric ligation clip includes first and second jaws that support a latch mechanism. The latch mechanism includes a latch member that is supported on one end of the first jaw and a latch member receiver that is supported on one end of the second jaw. The latch member and the latch member receiver are movable into engagement with each other when the ligation clip is moved to a clamped position to secure the ligation clip in the clamped position. The latch mechanism is configured to facilitate closure of the ligation clip with less force than required for closure of existing polymeric ligation clips. In addition, the latch mechanism is configured to provide more secure closure of the ligation clip to minimize a likelihood of inadvertent opening of the ligation clip during use.

## Description

### FIELD

This disclosure is directed to ligation clips for sealing body vessels and, more particularly, to polymeric ligation clips that include latch mechanisms for retaining jaws of the ligation clips in clamped positions.

### BACKGROUND

Polymeric ligation clips are well known in the surgical arts and are commonly used during a variety of surgical procedures to ligate tissue, e.g., a body vessel. Typically, ligation clips include first and second jaws that include clamping surfaces. The first and second jaws are pivotably connected to each other and movable between open and clamped positions. Typically, each of the first and second jaws includes a portion of a latching mechanism. When the ligation clip is moved to the clamped position and clamped about tissue, the portions of the latching mechanism engage to retain the ligation clip in the clamped position about the tissue.

In existing polymeric ligation clips, the portions of the latching mechanism on the first and second jaws engage each other as the ligation clip is moved from the open position to the clamped position. Upon engagement, one portion of the latching mechanism is deformed outwardly of and passes over the second portion of the latching mechanism as the ligation clip moves toward the clamped position. When the first portion of the latching mechanism passes over the second portion of the latching mechanism, the first portion returns to its non-deformed state to secure the ligation clip in the clamped position. In such a latching mechanism, a latching force required to deform the first portion of the latch mechanism outwardly of the second portion of the latch mechanism may be undesirably high making it difficult for a clinician to apply the ligation clip to tissue. Similarly, an unlatching force may be undesirably low to allow the ligation clip to be inadvertently moved to the open position and removed from the tissue.

### SUMMARY

One aspect of the disclosure is directed to a ligation clip that includes a first jaw, a second jaw, and a hinge portion. The first jaw defines a first clamping surface and has a distal portion and a proximal portion. The distal portion of the first jaw supports a latch member including a first contact surface and a first retention surface. The second jaw defines a second clamping surface and has a distal portion and a proximal portion. The distal portion of the second jaw has a latch member receiver including a second contact surface and a second retention surface. The hinge portion couples the proximal portion of the first jaw to the proximal portion of the second jaw to facilitate pivotal movement of the second jaw in relation to the first jaw from an open position to a clamped position. In the clamped position the first retention surface is engaged with the second retention surface. The first retention surface extends in a proximal direction towards the first clamping surface of the first jaw, and the second contact surface is linear and engages the first contact surface of the latch member as the ligation clip is moved towards the clamped position to deflect the latch member distally outwardly of the latch member receiver to guide the first retention surface into engagement with the second retention surface.

In aspects of the disclosure, the first retention surface is linear and defines a first plane that intersects the first clamping surface.

In some aspects of the disclosure, the first plane intersects a central portion of the first clamping surface.

In certain aspects of the disclosure, the second retention surface is linear and defines a second plane that is substantially parallel to the first plane when the ligation clip is in the clamped position.

In aspects of the disclosure, the first contact surface is a linear.

In some aspects of the disclosure, the first retention surface is a curved surface.

In certain aspects of the disclosure, the latch member includes a hook-shaped body, wherein the retention surface is formed on an inner surface of the hook-shaped body and the contact surface is formed on an outer surface of the hooked-shaped body.

In aspects of the disclosure, the distal portion of each of the first and second jaws includes bosses that are configured to engage a clip applier.

In aspects of the disclosure, the bosses on the distal portion of the second jaw are positioned on opposite sides of the second contact surface to define a channel with the second contact surface.

Another aspect of the disclosure is directed to a ligation clip that includes a first jaw, a second jaw, and a hinge portion. The first jaw defines a first clamping surface and has a distal portion and a proximal portion. The distal portion of the first jaw supports a latch member including a first contact surface and a first retention surface. The second jaw defines a second clamping surface and has a distal portion and a proximal portion. The distal portion of the second jaw has a latch member receiver including a second contact surface and a second retention surface. The hinge portion couples the proximal portion of the first jaw to the proximal portion of the second jaw to facilitate pivotal movement of the second jaw in relation to the first jaw from an open position to a clamped position. In the clamped position, the first retention surface is engaged with the second retention surface. The second contact surface is linear and engages the first contact surface of the latch member as the ligation clip is moved towards the clamped position to deflect the latch member distally outwardly of the latch member receiver to guide the first retention surface into engagement with the second retention surface.

In aspects of the disclosure, the first retention surface extends in a proximal direction towards the first clamping surface of the first jaw.

In some aspects of the disclosure, the first retention surface is linear and defines a first plane that intersects the first clamping surface.

Another aspect of the disclosure is directed to a ligation clip that includes a first jaw, a second jaw, and a hinge portion. The first jaw defines a first clamping surface and has a distal portion and a proximal portion. The distal portion of the first jaw supports a latch member including a first contact surface and a first retention surface. The second jaw defines a second clamping surface and has a distal portion and a proximal portion. The distal portion of the second jaw has a latch member receiver including a second contact surface and a second retention surface. The hinge portion couples the proximal portion of the first jaw to the proximal portion of the second jaw to facilitate pivotal movement of the second jaw in relation to the first jaw from an open position to a clamped position. In the clamped position the first retention surface is engaged with the second retention surface. The first retention surface extends in a proximal direction towards the first clamping surface of the first jaw.

In aspects of the disclosure, the first and second contact surfaces are linear and the second contact surface engages the first contact surface as the ligation clip is moved towards the clamped position to deflect the latch member distally outwardly of the latch member receiver to guide the first retention surface into engagement with the second retention surface.

In some aspects of the disclosure, the first retention surface of the latch member is linear and defines a first plane that intersects the first clamping surface of the first jaw.

### BRIEF DESCRIPTION OF THE DRAWINGS

Various exemplary aspects of the disclosed ligation clip are described herein below with reference to the drawings, wherein:
FIG. 1 is a side perspective view from a distal end of an exemplary aspect of the disclosed polymeric ligation clip in an open position;
FIG. 2 is an enlarged view of a first latch portion of the polymeric ligation clip shown in FIG. 1;
FIG. 3 is an enlarged view of a second latch portion of the polymeric ligation clip shown in FIG. 1;
FIG. 4 is a side perspective view of the polymeric ligation clip shown in FIG. 1 in a closed position;
FIG. 5 is a cross-sectional view taken along section line 5-5 of FIG. 4;
FIG. 6 is a cross-sectional view taken along section line 6-6 of FIG. 4; and
FIG. 7 is a cross-sectional view of taken through a latch mechanism of another exemplary embodiment of the disclosed polymeric ligation clip.

### DETAILED DESCRIPTION OF EMBODIMENTS

The disclosed polymeric ligation clip will now be described in detail with reference to the drawings in which like reference numerals designate identical or corresponding elements in each of the several views. It is to be understood that the disclosed aspects of the ligation clip are merely exemplary of the disclosure and may be embodied in various forms. Well-known functions or constructions are not described in detail to avoid obscuring the disclosure in unnecessary detail. Specific structural and functional details disclosed herein are not to be interpreted as limiting, but merely as a basis for the claims and as a representative basis for teaching one skilled in the art to variously employ the disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "clinician" is used generally to refer to medical personnel including doctors, nurses, and support personnel.

As used herein, the term "parallel" is understood to include relative configurations that are substantially parallel up to about +/- 10 degrees from true parallel.

The disclosed polymeric ligation clip includes first jaw and second jaws which support a latch mechanism. The latch mechanism includes a latch member that is supported on one end of the first jaw and a latch member receiver that is supported on one end of the second jaw. The latch member and the latch member receiver are movable into engagement with each other when the ligation clip is moved to a clamped position to secure the ligation clip in the clamped position. The latch member and the latch member receiver are configured to facilitate closure of the ligation clip with less force than required for closure of existing polymeric clips. In addition, the latch member and the latch member receiver are configured to provide a more secure closure of the ligation clip to minimize the likelihood of inadvertent movement of the ligation clip from the clamped position to the open position.

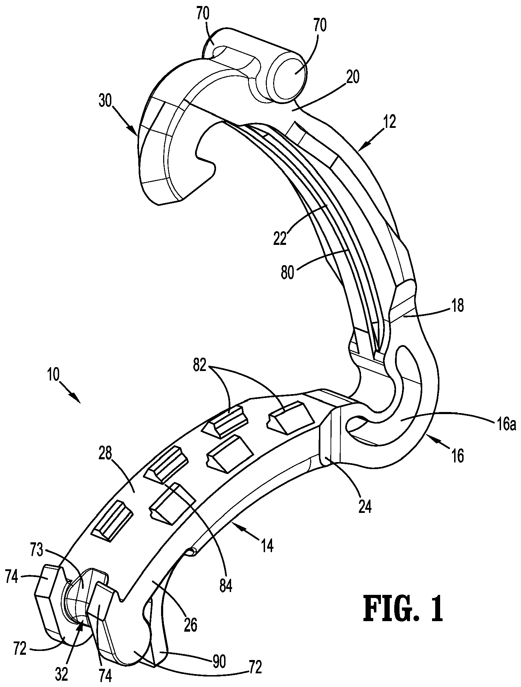

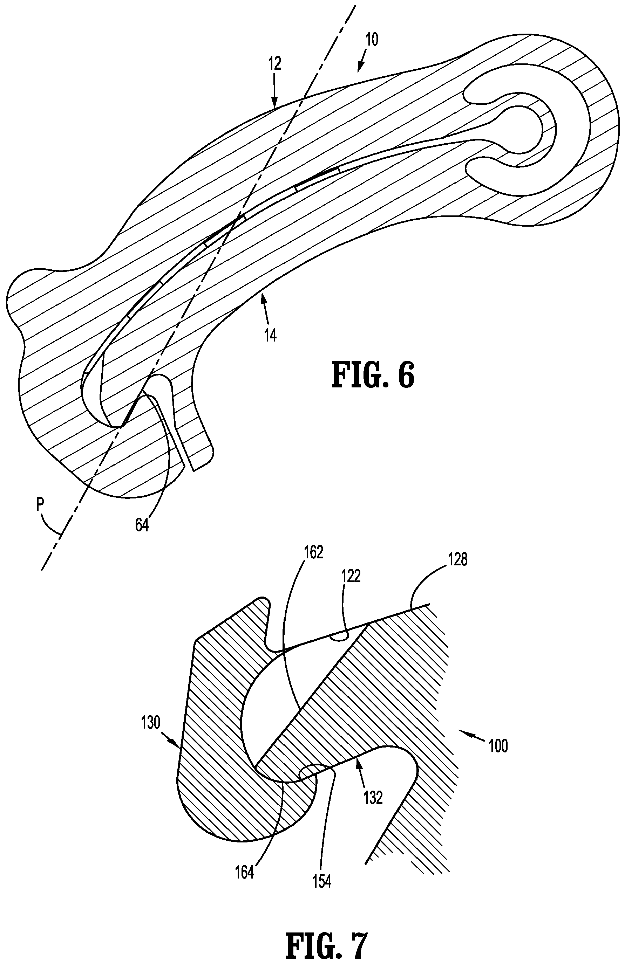

FIG. 1 illustrates exemplary aspects of the disclosed polymeric ligation clip which is shown generally as ligation clip 10. The ligation clip 10 includes a first jaw 12, a second jaw 14, and a hinge portion 16 coupling the first jaw 12 to the second jaw 14. The first jaw 12 is pivotable in relation to the second jaw 14 about the hinge portion 16 such that the ligation clip 10 is movable between an open position (FIG. 1) and a clamped position (FIG. 6). In embodiments, the first and second jaws 12, 14 are curved along their longitudinal axes such that the ligation clip 10 is arc-shaped in the clamped position (FIG. 6). Alternately, although other jaw configurations are envisioned. In aspects of the disclosure, the hinge portion 16 is integrally formed with the first and second jaws 12, 14, e.g., a living hinge, and defines a through bore 16a. The through bore 16a facilitates movement of the first jaw 12 in relation to the second jaw 14 between the open and clamped positions and allows for substantially complete closure of the proximal portions of the first and second jaws 12, 14. In certain aspects of the disclosure, the through bore 16a defines a crescent shape although other configurations are envisioned.

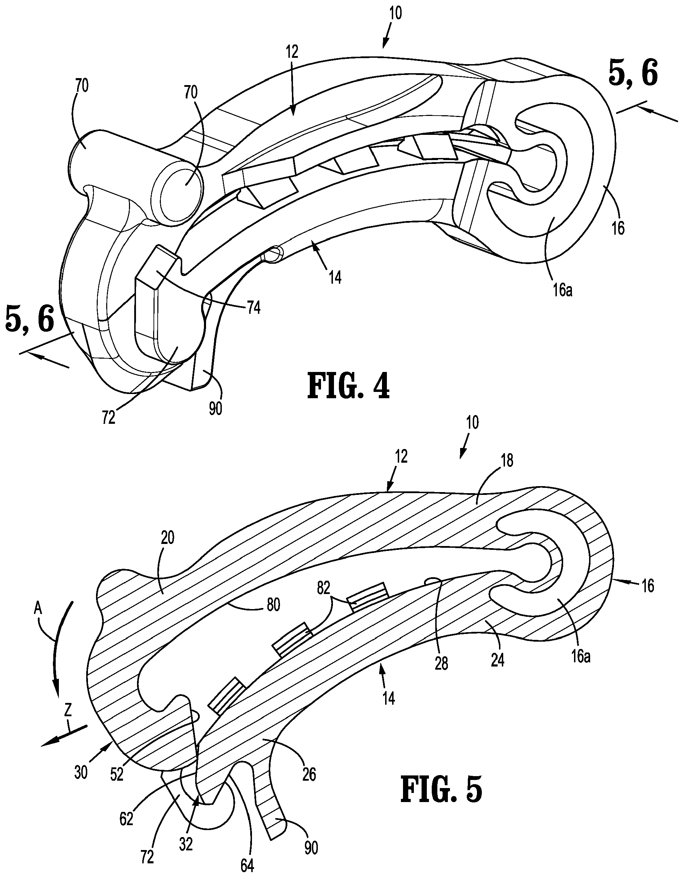

The first jaw 12 includes a proximal portion 18, a distal portion 20, and a clamping surface 22. The second jaw 14 includes a proximal portion 24, a distal portion 26, and a clamping surface 28. The proximal portions 18, 24 of the first and second jaws 12, 14, respectively, are coupled to the hinge portion 16. In the clamped position (FIG. 4), the first clamping surface 22 of the first jaw 12 is in juxtaposed alignment with the second clamping surface 28 of the second jaw 14.

The distal portions 20, 26 of the first and second jaws 12, 14, respectively, define a latching mechanism. More specifically, the distal portion 20 of the first jaw 12 includes a latch member 30 and the distal portion of the second jaw 14 defines a latch member receiver 32. The latch member 30 is moved into engagement with the latch member receiver 32 when the ligation clip 10 is moved to the clamped position to retain the ligation clip 10 in the clamped position as described below.

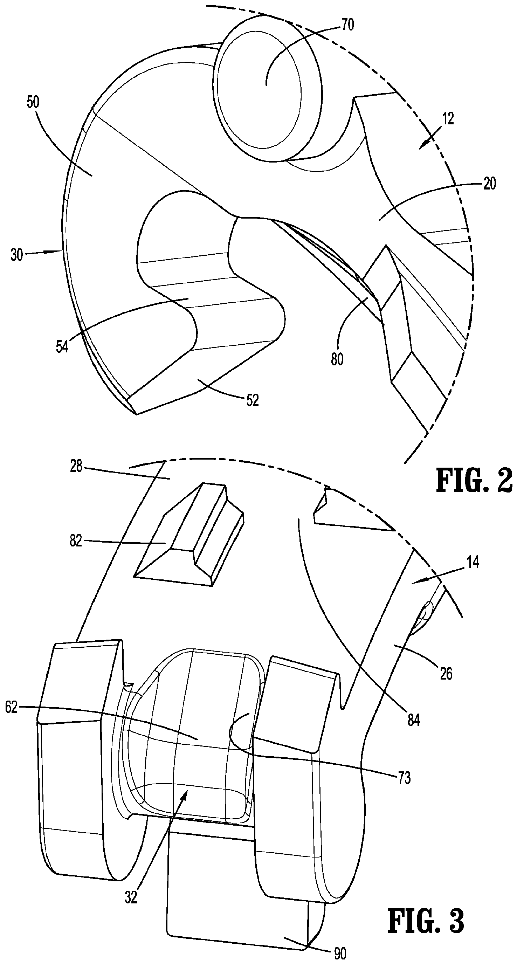

FIG. 2 illustrates an enlarged view of the latch member 30 which includes a hook-shaped body 50 having a first contact surface 52 and a first retention surface 54. The first contact surface 52 is positioned on an external surface of the hook-shaped body 50 and engages the latch member receiver 32 when the ligation clip 10 is moved towards the clamped position to deflect the latch member 30 outwardly in the direction of arrow "Z" in FIG. 5 past the latch member receiver 32. In aspects of the disclosure, the contact surface 52 of the latch member 30 is substantially linear or flat to minimize a force required to move the ligation clip 10 to the clamped position. The retention surface 54 is positioned on an internal surface of the hook-shaped body 50 and includes a substantially linear surface that defines a plane that intersects the clamping surface 22 of the first jaw 12. As used herein, "substantially" means 90 to 110 percent of the stated parameter.

FIG. 3 illustrates the latch member receiver 32 of the second jaw 14. The latch member receiver 32 includes a second contact surface 62 and a retention surface 64 (FIG. 5). The contact surface 62 is substantially linear and is positioned to engage the linear contact surface 52 of the first jaw 12 as the ligation clip 10 is moved towards the clamped position (FIG. 5) to guide the retention surface 54 of the latch member 30 into engagement with the retention surface 64 of the latch member receiver 32. Since the contact surfaces 52 and 62 are linear as compared to round or circular in existing ligation clips, the latch member 30 is deformed outwardly to a lesser degree to minimize the force required to move the ligation clip 10 to the clamped position. In certain aspects of the disclosure, the retention surface 64 of the latch member receiver 32 is also substantially linear and defines a plane that intersects the first clamping surface 22 of the first jaw 12 when the ligation clip 10 is in the clamped position. The retention surface 64 of the latch member receiver 32 engages the retention surface 54 of the latch member 30 when the ligation clip 10 is in the clamped position to provide a resistance to movement of the ligation clip 10 back to the open position. The angle defined by the first and second retention surfaces 54, 64 is steeper than the angle found in existing ligation clips to increase the force necessary to unlatch the ligation clip 10. More specifically, the orientation and configuration of the retention surfaces 54 and 64 of the first and second jaws 12, 14 provides an improved latching force than that found in existing ligation clips to increase the force required to unlatch the ligation clip 10.

In some aspects of the disclosed ligation clip 10, the angle, length, and/or width of the retention surfaces 54, 64 of the latch member 30 and latch member receiver 32, respectively, can be varied to increase or decrease the latching force of the latching mechanism of the ligation clip 10. In addition, the configuration of the retention surfaces 54, 64 may also be modified to adjust the latching force of the latching mechanism of the ligation clip 10. For example, the retention surfaces 54, 64 need not be linear but rather may be multi-linear, arcuate, or some combination of linear and arcuate.

FIGS. 1-4 illustrate the distal portions 20, 26 of the first and second jaws 12, 14, respectively. In aspects of the disclosure, the distal portion 20 of the first jaw 12 supports spaced bosses 70 that extend transversely outwardly from each side of the first jaw 12. The spaced bosses 70 are provided to engage a jaw of a clip applier (not shown) to retain the ligation clip 10 on the clip applier during application of the ligation clip 10 to tissue. Similarly, the distal portion 26 of the second jaw 14 also includes spaced bosses 72 which provide the same function as spaced bosses 70. The bosses 72 are positioned on opposite sides of contact surface 62 of the latch member receiver 32 such that the contact surface 62 of the latch member receiver 32 and the bosses 72 define a channel 73 between the bosses 72 which guides the latch member 30 into engagement with the latch member receiver 32 when the ligation clip 10 is moved to the clamped position. The distal portion 26 of the second jaw also includes spaced teeth 74 that grip and stretch tissue during movement of the ligation clip 10 to the clamped position to improve ligation characteristics of the ligation clip 10.

In certain aspects of the disclosed ligation clip 10 as illustrated in FIG. 1, the first and second clamping surfaces 22, 28 of the first and second jaws 12, 14, respectively, include tissue retention features to minimize slippage of the ligation clip 10 along tissue. For example, the first clamping surface 22 may include an elongated rib 80 and the second clamping surface 28 may include a plurality of protrusions 82 that are positioned on opposite sides of the second clamping surface to define a central channel 84 along the second clamping surface 28. When the ligation clip 10 is moved to the clamped position (FIG. 4), the longitudinal rib 80 is received within the central channel 84 to compress tissue between the rib 80 and the protrusions 82. It is envisioned that retention features on each of the first and second clamping surfaces may assume a variety of configurations to minimize slippage of the ligation clip 10 along tissue. Such retention features may include protrusions and/or recesses of a variety of configurations as is known in the art. In addition, the clamping surfaces 22, 28 of the ligation clip 10 may include a slip resistant coating or be formed of a slip resistant material.

The second jaw 14 supports a spring arm 90 that positioned to engage and be deflected by the latch member 30 to create a compressive force within the spring arm 90 that presses the latch member 30 into engagement with the latch member receiver 32 when the ligation clip 100 is in the clamped position. This compression helps to retain the latch member 30 and the latch member receiver 32 in the latched position.

In embodiments, the ligation clips described above may be made, in whole or in part, of a resilient bioabsorbable and/or biocompatible polymeric material. Examples of suitable bioabsorbable and/or biocompatible polymers include acetal polyoxymethylene (POM), polyethylene terephthalate (PET), polybutylene terephthalate (PBT), polyoxymethylene, polyetheretherketone (PEEK), polypropylene, and polyethylene or other thermoplastic materials having similar properties that can be injection-molded. The clip may also be made of a polymer material or materials in combination with radiolucent metal alloys. Alternately, other materials may be used to form the clip 10 including biocompatible metals, plastics and composites.

FIGS. 5 and 6 illustrate the ligation clip 10 as the ligation clip 10 moves from a partially clamped position (FIG. 5) to the clamped position (FIG. 6) in the direction indicated by arrow "A" in FIG. 5. As the first jaw 12 pivots toward the second jaw 14, the first contact surface 52 on the latch member 30 engages and moves along the second contact surface 62 of the latch member receiver 32. As the latch member 30 moves over the latch member receiver 32, the latch member 30 is deformed outwardly (in a distal direction) indicated by arrow "Z" in FIG. 5 and passes over the latch member receiver 32. When the latch member 30 passes over the latch member receiver 32, the latch member 30 returns to its undeformed state and the first retention surface 54 moves into engagement with the second retention surface 64 (FIG. 6).

As illustrated, the first and second retention surfaces 54, 64 are substantially parallel to each other and define a plane "P" (FIG. 6) that extends upwardly in the direction of the hinge portion 16 (in the proximal direction as used herein) and intersects the first and second clamping surfaces 22, 28, respectively. In certain aspects of the disclosure, the orientation of the plane "P" defined by the first and second retention surfaces 54, 64 is selected to intersect a central portion of the first and second clamping surfaces 22, 28, wherein the central portion of the first and second clamping surfaces 22, 28 is defined as a middle third of the length of the clamping surfaces 22, 28.

As summarized above, the linear configuration of the first and second contact surfaces 52, 62 of the latch member 30 and the latch member receiver 32 minimizes the force required to move the ligation clip 10 from the open position to the clamped position from that of existing ligation clips. In addition, the configuration and orientation of the retention surfaces 54, 64 of the latch member 30 and latch member receiver 30, 32, respectively, of the ligation clip 10 increases the force required to unclamp the ligation clip 10 from that of existing ligation clips.

FIG. 7 illustrates a distal portion of another aspect of the disclosed ligation clip shown generally as ligation clip 100. The ligation clip 100 is substantially as described above in regard to ligation clip 10 except that the configuration of the retention surfaces 154, 164, of the latch member 130 and the latch member receiver 132, respectively, are modified to define curved or arcuate retention surfaces 154, 164 rather than linear retention surfaces. The retention surface 154 of the latch member 130 defines a concavity. The retention surface 154 of the contact surface 162 of the latch member receiver 132 remains substantially linear. The curved retention surface 154 of the latch member 130 is angled upwardly in the proximal direction (towards the hinge portion) in a direction to intersect the clamping surfaces 122, 128 of the first and second jaws 112, 114 when the ligation clip 100 is in a clamped position. As with the ligation clip 10 (FIG. 1), the linear configuration of the contact surfaces 162 of the latch member 130 and the latch member receiver 132 minimizes the force required to move the ligation clip 100 from the open position to the clamped position (FIG. 7). In addition, the upwardly curved configuration and orientation of the retention surfaces 154, 164 of the latch member 130 and latch member receiver 132, respectively, of the ligation clip 10 increases the force required to unclamp the ligation clip 10.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:
1. A ligation clip comprising: a first jaw defining a first clamping surface and having a distal portion and a proximal portion, the distal portion supporting a latch member including a first contact surface and a first retention surface; a second jaw defining a second clamping surface and having a distal portion and a proximal portion, the distal portion having a latch member receiver including a second contact surface and a second retention surface; and a hinge portion coupling the proximal portion of the first jaw to the proximal portion of the second jaw, the hinge portion facilitating pivotal movement of the second jaw in relation to the first jaw from an open position to a clamped position, wherein in the clamped position the first retention surface is engaged with the second retention surface; wherein the first retention surface extends in a proximal direction towards the first clamping surface of the first jaw, and the second contact surface is linear and engages the first contact surface of the latch member as the ligation clip is moved towards the clamped position to deflect the latch member distally outwardly of the latch member receiver to guide the first retention surface into engagement with the second retention surface.
2. The ligation clip of paragraph 1, wherein the first retention surface is linear and defines a first plane that intersects the first clamping surface.
3. The ligation clip of paragraph 2, wherein the first plane intersects a central portion of the first clamping surface.
4. The ligation clip of paragraph 2, wherein the second retention surface is linear and defines a second plane that is substantially parallel to the first plane when the ligation clip is in the clamped position.
5. The ligation clip of paragraph 4, wherein the first contact surface is a linear.
6. The ligation clip of paragraph 1, wherein the first retention surface is a curved surface.
7. The ligation clip of paragraph 1, wherein the latch member includes a hook-shaped body, and the retention surface is formed on an inner surface of the hook-shaped body and the contact surface is formed on an outer surface of the hooked-shaped body.
8. The ligation clip of paragraph 1, wherein the distal portion of each of the first and second jaws includes bosses that are configured to engage a clip applier.
9. The ligation clip of paragraph 8, wherein the bosses on the distal portion of the second jaw are positioned on opposite sides of the second contact surface to define a channel with the second contact surface.
10. A ligation clip comprising: a first jaw defining a first clamping surface and having a distal portion and a proximal portion, the distal portion supporting a latch member including a first contact surface and a first retention surface; a second jaw defining a second clamping surface and having a distal portion and a proximal portion, the distal portion having a latch member receiver including a second contact surface and a second retention surface; and a hinge portion coupling the proximal portion of the first jaw to the proximal portion of the second jaw, the hinge portion facilitating pivotal movement of the second jaw in relation to the first jaw from an open position to a clamped position, wherein in the clamped position the first retention surface is engaged with the second retention surface; wherein the second contact surface is linear and engages the first contact surface of the latch member as the ligation clip is moved towards the clamped position to deflect the latch member distally outwardly of the latch member receiver to guide the first retention surface into engagement with the second retention surface.
11. The ligation clip of paragraph 10, wherein the first retention surface extends in a proximal direction towards the first clamping surface of the first jaw.
12. The ligation clip of paragraph 11, wherein the first retention surface is linear and defines a first plane that intersects the first clamping surface.
13. The ligation clip of paragraph 10, wherein the first plane intersects a central portion of the first clamping surface.
14. The ligation clip of paragraph 13, wherein the second retention surface is linear and defines a second plane that is substantially parallel to the first plane when the ligation clip is in the clamped position.
15. The ligation clip of paragraph 10, wherein the first contact surface is a linear.
16. The ligation clip of paragraph 10, wherein the first and second retention surfaces are curved.
17. A ligation clip comprising: a first jaw defining a first clamping surface and having a distal portion and a proximal portion, the distal portion supporting a latch member including a first contact surface and a first retention surface; a second jaw defining a second clamping surface and having a distal portion and a proximal portion, the distal portion having a latch member receiver including a second contact surface and a second retention surface; and a hinge portion coupling the proximal portion of the first jaw to the proximal portion of the second jaw, the hinge portion facilitating pivotal movement of the second jaw in relation to the first jaw from an open position to a clamped position, wherein in the clamped position the first retention surface is engaged with the second retention surface; wherein the first retention surface extends in a proximal direction towards the first clamping surface of the first jaw.
18. The ligation clip of paragraph 17, wherein the first and second contact surfaces are linear and the second contact surface engages the first contact surface as the ligation clip is moved towards the clamped position to deflect the latch member distally outwardly of the latch member receiver to guide the first retention surface into engagement with the second retention surface.
19. The ligation clip of paragraph 18, wherein the first retention surface of the latch member is linear and defines a first plane that intersects the first clamping surface of the first jaw.
20. The ligation clip of paragraph 19, wherein the second retention surface of the latch member is linear and defines a second plane that is substantially parallel to the first plane.

## Claims

1. A ligation clip comprising:
a first jaw defining a first clamping surface and having a distal portion and a proximal portion, the distal portion supporting a latch member including a first contact surface and a first retention surface;
a second jaw defining a second clamping surface and having a distal portion and a proximal portion, the distal portion having a latch member receiver including a second contact surface and a second retention surface; and
a hinge portion coupling the proximal portion of the first jaw to the proximal portion of the second jaw, the hinge portion facilitating pivotal movement of the second jaw in relation to the first jaw from an open position to a clamped position, wherein in the clamped position the first retention surface is engaged with the second retention surface;
wherein the first retention surface extends in a proximal direction towards the first clamping surface of the first jaw, and the second contact surface is linear and engages the first contact surface of the latch member as the ligation clip is moved towards the clamped position to deflect the latch member distally outwardly of the latch member receiver to guide the first retention surface into engagement with the second retention surface.

2. The ligation clip of claim 1, wherein the first retention surface is linear and defines a first plane that intersects the first clamping surface.

3. The ligation clip of claim 2, wherein the first plane intersects a central portion of the first clamping surface; and/or wherein the second retention surface is linear and defines a second plane that is substantially parallel to the first plane when the ligation clip is in the clamped position.

4. The ligation clip of claim 3, wherein the first contact surface is a linear.

5. The ligation clip of any preceding claim, wherein the first retention surface is a curved surface; and/or wherein the latch member includes a hook-shaped body, and the retention surface is formed on an inner surface of the hook-shaped body and the contact surface is formed on an outer surface of the hooked-shaped body.

6. The ligation clip of any preceding claim, wherein the distal portion of each of the first and second jaws includes bosses that are configured to engage a clip applier; preferably wherein the bosses on the distal portion of the second jaw are positioned on opposite sides of the second contact surface to define a channel with the second contact surface.

7. A ligation clip comprising:
a first jaw defining a first clamping surface and having a distal portion and a proximal portion, the distal portion supporting a latch member including a first contact surface and a first retention surface;
a second jaw defining a second clamping surface and having a distal portion and a proximal portion, the distal portion having a latch member receiver including a second contact surface and a second retention surface; and
a hinge portion coupling the proximal portion of the first jaw to the proximal portion of the second jaw, the hinge portion facilitating pivotal movement of the second jaw in relation to the first jaw from an open position to a clamped position, wherein in the clamped position the first retention surface is engaged with the second retention surface;
wherein the second contact surface is linear and engages the first contact surface of the latch member as the ligation clip is moved towards the clamped position to deflect the latch member distally outwardly of the latch member receiver to guide the first retention surface into engagement with the second retention surface.

8. The ligation clip of claim 7, wherein the first retention surface extends in a proximal direction towards the first clamping surface of the first jaw; preferably wherein the first retention surface is linear and defines a first plane that intersects the first clamping surface.

9. The ligation clip of claim 7 or claim 8, wherein the first plane intersects a central portion of the first clamping surface.

10. The ligation clip of claim 9, wherein the second retention surface is linear and defines a second plane that is substantially parallel to the first plane when the ligation clip is in the clamped position.

11. The ligation clip of any of claims 7 to 10, wherein the first contact surface is a linear.

12. The ligation clip of any of claims 7 to 11, wherein the first and second retention surfaces are curved.

13. A ligation clip comprising:
a first jaw defining a first clamping surface and having a distal portion and a proximal portion, the distal portion supporting a latch member including a first contact surface and a first retention surface;
a second jaw defining a second clamping surface and having a distal portion and a proximal portion, the distal portion having a latch member receiver including a second contact surface and a second retention surface; and
a hinge portion coupling the proximal portion of the first jaw to the proximal portion of the second jaw, the hinge portion facilitating pivotal movement of the second jaw in relation to the first jaw from an open position to a clamped position, wherein in the clamped position the first retention surface is engaged with the second retention surface;
wherein the first retention surface extends in a proximal direction towards the first clamping surface of the first jaw.

14. The ligation clip of claim 13, wherein the first and second contact surfaces are linear and the second contact surface engages the first contact surface as the ligation clip is moved towards the clamped position to deflect the latch member distally outwardly of the latch member receiver to guide the first retention surface into engagement with the second retention surface; preferably wherein the first retention surface of the latch member is linear and defines a first plane that intersects the first clamping surface of the first jaw.

15. The ligation clip of claim 14, wherein the second retention surface of the latch member is linear and defines a second plane that is substantially parallel to the first plane.
